# EUROPEAN PATENT APPLICATION

(11) **EP 4 803 538 A1**
(43) Date of publication of application: **09.09.2026**
(21) Application number: 25893264.9
(22) Date of filing: 22.12.2025
(51) Int. Cl.: C07K 16/18, G01N 33/569, G01N 33/577, A61K 39/395, A61P 31/22, A61P 35/00, C12N 15/13

(54) **MONOCLONAL ANTIBODY 5E9 FOR RECOGNIZING HUMAN R9AP AND USE THEREOF**

(30) Priority: 25.12.2024 CN 202411919552
(71) Applicant: Sun Yat-Sen University Cancer Center (SYSUCC), Guangzhou Guangdong 510060 (CN)
(72) Inventor: ZENG, Musheng, Guangzhou, Guangdong 510060 (CN); SUN, Cong, Guangzhou, Guangdong 510060 (CN); XIE, Chu, Guangzhou, Guangdong 510060 (CN); WU, Peihuang, Guangzhou, Guangdong 510060 (CN)
(74) Representative: Gassner, Birgitta
(86) International application number: PCT/CN2025/144284
(87) International publication number: WO 2026/138728

(57) **Abstract**

The present disclosure belongs to the technical field of antibody, and discloses a monoclonal antibody 5E9 recognizing human R9AP protein and use thereof. The monoclonal antibody or antigen-binding fragment thereof has high affinity for R9AP protein and can significantly inhibit EBV infection of epithelial cells and B cells. The antibody can be used for detecting the presence or level of R9AP protein in a sample, detecting a disease caused by high expression of R9AP, and preventing EBV infection and/or treating and/or preventing a disease caused by EBV infection.

## Description

### TECHNICAL FIELD

The present disclosure belongs to the technical field of antibody, and specifically relates to a monoclonal antibody 5E9 recognizing human R9AP protein and use thereof.

### BACKGROUND

R9AP protein (Retinal G protein-coupled receptor interacting protein, R9AP) is a cell membrane protein that mainly plays an important role in retinal photoreceptor cells. This protein is encoded by R9AP gene and participates in rhodopsin-related signal transduction process within a cell.

The main function of the R9AP protein is to regulate the function of rhodopsin in the retina. The R9AP protein interacts with the G protein-coupled receptor complex of rhodopsin, helping to maintain the stable connection between rhodopsin and G protein, thereby facilitating the transmission of visual signals. Particularly during the photoreception process, rhodopsin is activated by light, triggering a series of signal transduction reactions, and R9AP protein is an indispensable part of this process. At the molecular level, R9AP interacts with rhodopsin and its related molecules (such as G protein) to ensure that visual signals are rapidly and effectively transmitted from external light stimuli to the nervous system, resulting in visual perception. Deficiency or dysfunction of R9AP may lead to impaired visual function and even cause some hereditary eye diseases. Notably, the expression of R9AP is also associated with the occurrence of various tumors, and it may play a potential role in the occurrence and development of certain tumors. Specifically, the relationship between high R9AP expression and the following tumor types has been preliminarily explored: 1. Breast cancer: Some studies have shown that R9AP may have a high expression level in breast cancer cells, especially in certain advanced or invasive breast cancers cells, and high expression of R9AP may be associated with tumor cell proliferation, migration, and invasiveness. 2. Ovarian cancer: R9AP has also been found to be highly expressed in ovarian cancer cells, and its high expression may be related to the survival and drug resistance of ovarian cancer cells. This high expression may promote tumor cell growth by regulating signal pathways related to cell survival and growth. 3. Colorectal cancer: In colorectal cancer, some studies have found that R9AP expression level is high and is associated with tumor malignancy and metastatic potential. R9AP may influence the growth and migration of colorectal cancer cells by participating in G protein-coupled receptor signaling. 4. Lung cancer: R9AP is also highly expressed in certain types of lung cancer, especially non-small cell lung cancer. This high expression may be associated with drug resistance, proliferation, and metastatic ability of tumor. 5. Gastric cancer: In gastric cancer research, high expression of R9AP protein is considered to be associated with aggressive behavior and poor prognosis of gastric cancer. 6. Liver cancer: In some studies for liver cancer, high expression of R9AP has also been observed, which may be related to characteristics such as growth, division, and drug resistance of liver cancer cells.

Moreover, recent studies have also found that R9AP is an important common receptor for Epstein-Barr virus (EBV) infection of cells, participating in both infection processes of B cells and epithelial cells by the virus. Therefore, an antibody against R9AP can inhibit EBV recognition of R9AP, thereby inhibiting further virus infection of cells.

Currently, there is no effective vaccine against EBV, and diseases caused by EBV infection also lack specific therapeutic approaches. The treatment of infectious mononucleosis mostly uses antiviral drugs such as acyclovir. Although these drugs can alleviate symptoms to some extent, they cannot eliminate EBV in B lymphocytes and the throat epithelium. Therefore, developing an antibody against R9AP can serve as an important therapeutic approach to block EBV infection.

### SUMMARY

A first aspect of the present disclosure aims to provide a monoclonal antibody or an antigen-binding fragment thereof.

A second aspect of the present disclosure aims to provide a recombinant protein.

A third aspect of the present disclosure aims to provide a biological material related to the monoclonal antibody or the antigen-binding fragment thereof according to the first aspect or the recombinant protein according to the second aspect of the present disclosure.

A fourth aspect of the present disclosure aims to provide a method for preparing the monoclonal antibody or the antigen-binding fragment thereof according to the first aspect or the recombinant protein according to the second aspect of the present disclosure.

A fifth aspect of the present disclosure aims to provide a conjugate.

A sixth aspect of the present disclosure aims to provide use of the monoclonal antibody or the antigen-binding fragment thereof according to the first aspect, the recombinant protein according to the second aspect, the biological material according to the third aspect, and/or the conjugate according to the fifth aspect of the present disclosure in the preparation of a product.

A seventh aspect of the present disclosure aims to provide a drug.

An eighth aspect of the present disclosure aims to provide a vaccine.

To achieve the above objectives, the technical solutions used in the present disclosure are as follows.

The first aspect of the present disclosure provides a monoclonal antibody or an antigen-binding fragment thereof against EBV R9AP, and the monoclonal antibody or the antigen-binding fragment thereof comprises a heavy chain variable region and a light chain variable region;
the heavy chain variable region comprises CDR-H1, CDR-H2, and CDR-H3;
the CDR-H1, CDR-H2, and CDR-H3 are CDR1, CDR2, CDR3 of an amino acid sequence shown in SEQ ID NO: 4;
the light chain variable region comprises CDR-L1, CDR-L2, and CDR-L3;
the CDR-L1, CDR-L2, and CDR-L3 are CDR1, CDR2, CDR3 of an amino acid sequence shown in SEQ ID NO: 15.

Preferably, amino acid sequences of the CDR-H1, CDR-H2, CDR-H3, CDR-L1, CDR-L2, CDR-L3 are sequentially as shown in SEQ ID NO: 5, SEQ ID NO: 6, SEQ ID NO: 7, SEQ ID NO: 16, SEQ ID NO: 17, and SEQ ID NO: 18, and the CDRs are defined by an IMGT definition scheme.

Preferably, amino acid sequences of the CDR-H1, CDR-H2, CDR-H3, CDR-L1, CDR-L2, CDR-L3 are sequentially as shown in SEQ ID NO: 5, SEQ ID NO: 6, SEQ ID NO: 7, SEQ ID NO: 16, SEQ ID NO: 17, and SEQ ID NO: 18, and the CDRs are defined by a Kabat definition scheme.

Preferably, amino acid sequences of the CDR-H1, CDR-H2, CDR-H3, CDR-L1, CDR-L2, CDR-L3 are sequentially as shown in SEQ ID NO: 8, SEQ ID NO: 9, SEQ ID NO: 10, SEQ ID NO: 16, SEQ ID NO: 17, and SEQ ID NO: 18, and the CDRs are defined by a Chothia definition scheme.

Preferably, amino acid sequences of the CDR-H1, CDR-H2, CDR-H3, CDR-L1, CDR-L2, CDR-L3 are sequentially as shown in SEQ ID NO: 11, SEQ ID NO: 12, SEQ ID NO: 13, SEQ ID NO: 19, SEQ ID NO: 20, and SEQ ID NO: 21, and the CDRs are defined by a Contact definition scheme.

Preferably, an amino acid sequence of the heavy chain variable region comprises:
a1) SEQ ID NO: 4; or
a2) an amino acid sequence obtained by substituting and/or deleting and/or adding one or more amino acids in SEQ ID NO: 4, and having the same function as the protein shown in SEQ ID NO: 4; or
a3) an amino acid sequence having 99%, 98%, 97%, 96%, 95%, 94%, 93%, 92%, 91%, 90%, 89%, 88%, 87%, 86%, 85%, 84%, 83%, 82%, 81% or 80% homology with SEQ ID NO: 4, and having the same function as the protein shown in SEQ ID NO: 4;
an amino acid sequence of the light chain variable region comprises:
   b1) SEQ ID NO: 15; or
   b2) an amino acid sequence obtained by substituting and/or deleting and/or adding one or more amino acids in SEQ ID NO: 15, and having the same function as the protein shown in SEQ ID NO: 15; or
   b3) an amino acid sequence having 99%, 98%, 97%, 96%, 95%, 94%, 93%, 92%, 91%, 90%, 89%, 88%, 87%, 86%, 85%, 84%, 83%, 82%, 81% or 80% homology with SEQ ID NO: 15, and having the same function as the protein shown in SEQ ID NO: 17.

Preferably, the monoclonal antibody or the antigen-binding fragment thereof comprises at least one of a full-length antibody, Fab, Fab', F(ab')2, Fv, scFv, a bispecific antibody, or a multispecific antibody.

Preferably, the heavy chain further comprises a heavy chain constant region; and/or, the light chain further comprises a light chain constant region.

Preferably, the heavy chain further comprises a heavy chain signal peptide; and/or, the light chain further comprises a light chain signal peptide.

Preferably, an amino acid sequence of the heavy chain signal peptide comprises:
e1) an amino acid sequence consisting of positions 1 to 19 of SEQ ID NO: 3; or
e2) an amino acid sequence obtained by substituting and/or deleting and/or adding one or more amino acids in the amino acid sequence of e1), and having the same function as the protein of the amino acid sequence of e1); or
e3) an amino acid sequence having 99%, 98%, 97%, 96%, 95%, 94% or 93% homology with the amino acid sequence of e1), and having the same function as the protein of the amino acid sequence of e1).

Preferably, an amino acid sequence of the light chain signal peptide comprises:
f1) an amino acid sequence consisting of positions 1 to 19 of SEQ ID NO: 14; or
f2) an amino acid sequence obtained by substituting and/or deleting and/or adding one or more amino acids in the amino acid sequence of f1), and having the same function as the protein of the amino acid sequence of f1); or
f3) an amino acid sequence having 99%, 98%, 97%, 96%, 95%, 94% or 93% homology with the amino acid sequence of f1), and having the same function as the protein of the amino acid sequence of f1).

Preferably, an amino acid sequence of the human R9AP protein comprises:
g1) SEQ ID NO: 1; or
g2) an amino acid sequence obtained by substituting and/or deleting and/or adding one or more amino acids in the amino acid sequence of g1), and having the same function as the protein of the amino acid sequence of g1); or
g3) an amino acid sequence having 99%, 98%, 97%, 96%, 95%, 94% or 93% homology with the amino acid sequence of g1), and having the same function as the protein of the amino acid sequence of g1).

The second aspect of the present disclosure provides a recombinant protein, comprising the monoclonal antibody or the antigen-binding fragment thereof according to the first aspect; and optionally a tag sequence that assists expression and/or purification.

Preferably, the tag sequence is at least one selected from the group consisting of a histidine (His) tag, a GGGS sequence (SEQ ID NO: 26), and a FLAG tag; more preferably a His tag; even more preferably a 6×His tag.

The third aspect of the present disclosure provides a biological material related to the monoclonal antibody or the antigen-binding fragment thereof according to the first aspect, or the recombinant protein according to the second aspect, wherein the biological material comprises at least one of h1) to h16):
h1) a nucleic acid molecule encoding the monoclonal antibody or the antigen-binding fragment thereof according to the first aspect, or the recombinant protein according to the second aspect;
h2) an expression cassette comprising the nucleic acid molecule of h1);
h3) a vector comprising the nucleic acid molecule of h1);
h4) a vector comprising the expression cassette of h2);
h5) a transgenic cell line comprising the nucleic acid molecule of h1);
h6) a transgenic cell line comprising the expression cassette of h2);
h7) a transgenic cell line comprising the vector of h3);
h8) a transgenic cell line comprising the vector of h4);
h9) a microorganism comprising the nucleic acid molecule of h1);
h10) a microorganism comprising the expression cassette of h2);
h11) a microorganism comprising the vector of h3);
h12) a microorganism comprising the vector of h4);
h13) a virus comprising the nucleic acid molecule of h1);
h14) a virus comprising the expression cassette of h2);
h15) a virus comprising the vector of h3);
h16) a virus comprising the vector of h4).

Preferably, the transgenic cell line does not comprise a propagation material.

Preferably, the nucleic acid molecule encoding the monoclonal antibody or the antigen-binding fragment thereof according to the first aspect comprises a nucleic acid molecule encoding the heavy chain of the monoclonal antibody or the antigen-binding fragment thereof according to the first aspect and a nucleic acid molecule encoding the light chain of the monoclonal antibody or the antigen-binding fragment thereof according to the first aspect.

The fourth aspect of the present disclosure provides a method for preparing the monoclonal antibody or the antigen-binding fragment thereof according to the first aspect or the recombinant protein according to the second aspect, the method comprises the following steps: expressing and then purifying the biological material according to the third aspect to obtain the monoclonal antibody or the antigen-binding fragment thereof or the recombinant protein.

The fifth aspect of the present disclosure provides a conjugate comprising at least one of the monoclonal antibody or the antigen-binding fragment thereof according to the first aspect of the present disclosure, or the recombinant protein according to the second aspect of the present disclosure; and a conjugated moiety, the conjugated moiety comprises at least one of a detectable label, a drug, a toxin, a cytokine, a radionuclide, or an enzyme.

Preferably, the detectable label is selected from the group consisting of a radioisotope, a fluorescent substance, a chemiluminescent substance, a colored substance, and any combination thereof.

Preferably, the conjugate is selected from the group consisting of a fluorescent substance, a chemiluminescent label, a colored substance, a radioisotope, a magnetic resonance imaging (MRI) or computed tomography (CT) contrast agent, an enzyme capable of producing a detectable product, a radionuclide, a biotoxin, a cytokine (e.g., IL-2, etc.), an antibody, an antibody Fc fragment, an antibody scFv fragment, gold nanoparticles/nanorods, viral particles, liposomes, magnetic nanoparticles, a prodrug-activating enzyme, a chemotherapeutic agent (e.g., cisplatin), and nanoparticles of any form.

The sixth aspect of the present disclosure provides use of the monoclonal antibody or the antigen-binding fragment thereof according to the first aspect, the recombinant protein according to the second aspect, the biological material according to the third aspect, and/or the conjugate according to the fifth aspect of the present disclosure in the preparation of a product;
the product comprises at least one of a drug, a reagent, a detection plate, a kit, or a detection chip.

Preferably, the drug has at least one of the following functions i1) to i2):
i1) preventing EBV infection;
i2) treating and/or preventing a disease caused by EBV infection.

Preferably, the reagent, detection plate, detection chip or kit has at least one of the following functions j1) to j2):
j1) detecting a presence or level of R9AP protein in a sample;
j2) detecting a disease associated with high expression of R9AP.

Preferably, the disease comprises at least one of breast cancer, ovarian cancer, colorectal cancer, lung cancer, gastric cancer, or liver cancer.

The seventh aspect of the present disclosure provides a product, and the product comprises at least one of k1) to k3):
k1) the monoclonal antibody or the antigen-binding fragment thereof according to the first aspect of the present disclosure;
k2) the recombinant protein according to the second aspect of the present disclosure;
k3) the conjugate according to the fifth aspect of the present disclosure;
the product comprises at least one of a reagent, a detection plate, a kit, or a detection chip.

Preferably, the reagent, detection plate, detection chip or kit has at least one of the following functions j1) to j2):
j1) detecting a presence or level of R9AP protein in a sample;
j2) detecting a disease associated with high expression of R9AP.

Preferably, the disease comprises at least one of breast cancer, ovarian cancer, colorectal cancer, lung cancer, gastric cancer, or liver cancer.

The eighth aspect of the present disclosure provides a drug, the drug comprises at least one of l1) to l4):
l1) the monoclonal antibody or the antigen-binding fragment thereof according to the first aspect of the present disclosure;
l2) the recombinant protein according to the second aspect of the present disclosure;
l3) the biological material according to the third aspect of the present disclosure;
l4) the conjugate according to the fifth aspect of the present disclosure.

Preferably, the drug further comprises a pharmaceutically acceptable carrier.

Preferably, the drug has at least one of the following functions i1) to i2):
i1) preventing EBV infection;
i2) treating and/or preventing a disease caused by EBV infection.

Preferably, the disease comprises at least one of nasopharyngeal carcinoma, gastric cancer, Hodgkin's lymphoma, Burkitt's lymphoma, NK/T cell lymphoma, lymphoproliferative disease, or infectious mononucleosis.

Preferably, the drug comprises a vaccine.

Preferably, provided is a vaccine, comprising at least one of l1) to l4) and an adjuvant:
l1) the monoclonal antibody or the antigen-binding fragment thereof according to the first aspect of the present disclosure;
l2) the recombinant protein according to the second aspect of the present disclosure;
l3) the biological material according to the third aspect of the present disclosure;
l4) the conjugate according to the fifth aspect of the present disclosure.

The beneficial effects of the present disclosure are as follows.

The present disclosure provides a monoclonal antibody or an antigen-binding fragment thereof against human R9AP protein. This monoclonal antibody or antigen-binding fragment thereof has high affinity for human R9AP protein, and can detect the expression level of human R9AP protein at multiple levels. Moreover, this monoclonal antibody or antigen-binding fragment thereof can significantly inhibit EBV infection of epithelial cells and B cells. Therefore, it can be used for detecting the presence or level of R9AP protein in a sample, detecting a disease caused by high expression of R9AP, and preventing EBV infection and/or treating and/or preventing a disease caused by EBV infection.

### BRIEF DESCRIPTION OF DRAWINGS

FIG. 1 is a graph showing the results of ELISA binding detection of monoclonal antibody 5E9 with R9AP protein.
FIG. 2 is a graph showing the results of BLI binding detection of monoclonal antibody 5E9 with R9AP protein.
FIG. 3 is a graph showing the results of WB-specific binding detection of monoclonal antibody 5E9 with R9AP protein.
FIG. 4 is a graph showing the neutralization results of EBV on epithelial cells by monoclonal antibody 5E9.
FIG. 5 is a graph showing the neutralization results of EBV on B cells by monoclonal antibody 5E9.

### DETAILED DESCRIPTION

The concept and technical effects of the present disclosure will be clearly and completely described below in conjunction with the examples to fully understand the objectives, features, and effects of the present disclosure. It is apparent that the described examples are only a part of the examples of the present disclosure, rather than all examples. Based on the examples of the present disclosure, other examples obtained by those skilled in the art without creative efforts shall fall within the protection scope of the present disclosure.

In the following examples, experimental methods without specified conditions are generally performed under conventional conditions or under conditions recommended by the manufacturer. The materials and reagents used in these examples, unless otherwise specified, are all commercially available reagents and materials.

### Example 1: Preparation of human R9AP protein, animal immunization, and antibody screening

### 1.1 Expression and purification of R9AP protein

### 1.1.1 Experimental materials

(1) Expression vector: eukaryotic expression vector pGEX-6P-1(+) (Addgene).
(2) Expression system: prokaryotic expression system: DE3 cells.
(3) Reagents and consumables: Terrific Broth (TB) medium (Hopebio), Glutathione S-transferase (GST) tag protein purification agarose beads (Cytiva), etc., and other conventional reagents and consumables were all purchased from commercial sources.
(4) Gene: The human R9AP gene was optimized and synthesized by Nanjing GenScript Biotechnology Co., Ltd., China and inserted into the pGEX-6P-1 vector to construct a recombinant plasmid, hereinafter referred to as GST-R9AP.

The recombinant protein of R9AP extracellular domain has a total length of 210 amino acid residues, with the specific sequence as follows:

MAREECKALLDGLNKTTACYHHLVLTVGGSADSQNLRQELQKTRQKAQELAVSTCA
RLTAVLRDRGLAADERAEFERLWVAFSGCLDLLEADMRRALELGAAFPLHAPRRPLVRTGV

AGASSGVAARALSTRSLRLEAEGDFDVADLRELEREVLQVGEMIDNMEMKVNVPRWTVQ
ARQAAGAELLSTVSAGPSSVVSLQERGGGCDPRK (SEQ ID NO: 1).

The full-length DNA expression sequence has 630 base pairs in total, with the specific sequence as follows:

ATGGCGAGGGAGGAGTGCAAGGCGCTGCTGGACGGGCTCAACAAGACGACTGCG
TGCTACCACCACCTGGTGCTGACCGTCGGTGGCTCGGCGGACTCGCAGAACCTGCGGC
AGGAGCTGCAAAAGACGCGCCAGAAGGCGCAGGAGCTGGCGGTGTCCACCTGCGCCC
GGCTGACTGCTGTGCTGCGCGACCGGGGCCTGGCCGCCGACGAGCGCGCCGAGTTCGA
GCGGCTCTGGGTGGCCTTCTCGGGCTGCCTGGACCTGTTGGAAGCGGACATGCGACGC
GCGCTGGAGCTGGGCGCCGCGTTCCCGCTGCACGCGCCGCGGCGGCCGCTGGTGCGCA
CAGGTGTGGCTGGCGCCTCCTCCGGCGTGGCGGCGCGCGCGCTGAGCACCCGCAGCCT
GCGGCTCGAGGCGGAGGGCGACTTCGACGTCGCGGACCTGCGGGAGCTGGAGCGCGA
GGTCCTTCAGGTGGGCGAGATGATCGACAACATGGAGATGAAGGTCAACGTGCCCCGC
TGGACCGTGCAAGCCCGGCAGGCGGCGGGCGCCGAGCTCCTGTCCACGGTCAGCGCCG
GCCCCTCCTCGGTCGTGTCCTTGCAGGAGCGCGGGGGGGGTTGCGACCCCAGGAAG
(SEQ ID NO: 2).

### 1.1.2 Protein expression

### Step 1: Transformation and picking of single clones

(1) The synthesized plasmid was added to thawed *Escherichia coli* DH5α, and was left to stand on ice for 5 minutes.
(2) A resulting bacterial liquid was placed in a shaking metal bath and was heat-shocked at 42 °C, 120 rpm for 60-90 seconds.
(3) A resulting bacterial liquid was left to stand on ice for cooling for 5 minutes.
(4) The bacterial liquid was spread onto an ampicillin-resistant TB medium plate using a sterile spreading rod, and was cultured overnight.
(5) On the next day, a single colony on the plate surface was picked using a sterile pipette tip and was transferred into 500 mL of medium, 0.1 mg/mL ampicillin was added to the medium, and a resulting mixture was cultured at 37 °C, 220 rpm for 12-16 hours.
(6) A resulting bacterial liquid was cultured for around 8 hours, an OD value was measured using a Nanodrop spectrophotometer until the OD value reached approximately 1.8-2.0.
(7) Conditions for the incubator were adjusted to 18 °C, 220 rpm for cooling the bacterial liquid.
(8) The bacterial liquid was added with 1 mM IPTG solution and culturing was continued for 8-12 hours.

### Step 2: Purification of prokaryotic cultured protein

(1) Bacterial liquid after the culturing was centrifuged at 4000 rpm, 4 °C for 10 minutes, a resulting bacterial pellet was collected, and the supernatant was discarded.
(2) The pellet was resuspended in a resuspension buffer (buffer, 250 mM NaCl, 50 mM HEPES pH 8.0, 2% Glycerol, 30 mM Imidazole, 1 mM DTT) at a ratio of 50 mL of the resuspension buffer per 1 L of the bacterial liquid.
(3) The resuspended pellet was broken by a pressure homogenizer three times at 4 °C, 800 Pa.
(4) The broken pellet was centrifuged at 15000g, 4 °C for 30 minutes, and the supernatant was separated.
(5) The supernatant was filtered twice through a 0.22 µm filter membrane.
(6) The supernatant was transferred to a GST beads purification column by a siphon method, and was allowed to completely pass through the column. If multiple recoveries were needed, the supernatant could be recovered at this step and purified again.
(7) The purification column was equilibrated with three column volumes of the resuspension buffer.
(8) Elution was performed with two column volumes of elution buffer (250 mM NaCl, 50 mM HEPES pH 8.0, 2% Glycerol, 20 mM GSH, 1 mM DTT), and a resulting eluate was collected.
(9) Samples of the bacterial lysate, centrifugal supernatant, post-centrifugation pellet, flow-through, equilibration buffer, and elution buffer were respectively retained, and protein expression and purification were verified by sodium dodecyl sulfate polyacrylamide gel electrophoresis (SDS-PAGE).
(10) The eluate was concentrated using an ultrafiltration tube with an appropriate molecular weight cut-off until the volume was reduced to less than 1 mL. The concentrated eluate was centrifuged at 12000g, 4 °C for 5 minutes to remove precipitate.
(11) The concentrated eluate was subjected to size exclusion chromatography using an AKTA purifier equipped with a Superdex200 Increase 10/300 GL column. Protein sample at the peak position of the size exclusion chromatography was collected, and was verified by SDS-PAGE together with the previous samples.

### 2.2.5 Protein storage

(1) After the purity of the protein was confirmed by SDS-PAGE, the concentration of the obtained concentrated protein was detected using a Bicinchoninic acid (BCA) kit. The brief steps were as follows.
(2) BSA standards were prepared. Starting from a concentration of 1 mg/mL, a 10-fold serial dilution was performed, and samples of 7 gradients were prepared.
(3) 0, 1, 2, 4, 8, 12, 16, and 20 µL of the standards were added respectively into standard wells of a 96-well plate, and were then made up to 20 µL with standard diluent, corresponding to standard concentrations of 0, 0.025, 0.05, 0.1, 0.2, 0.3, 0.4, and 0.5 mg/mL, respectively.
(4) An appropriate volume of sample was added to the sample wells of the 96-well plate. If the sample volume was less than 20 µL, it was made up to 20 µL with standard diluent. The sample volume was recorded.
(5) 200 µL of BCA working solution was added to each well, and the plate was placed at 37°C for 20-30 minutes.
(6) The absorbance of each well at a wavelength of 562 nm was measured using a microplate reader.
(7) A standard curve was plotted using the absorbance and concentrations of the standards, and the protein concentration was calculated by substituting the absorbance value of the sample well into the curve.
(8) After the protein concentration was determined, the protein was diluted to 1 mg/mL with buffer, and was aliquoted into 1.5 mL EP tubes. The protein was flash-frozen using liquid nitrogen, and was then stored for long-term in a -80 °C freezer. If immediate use was required, the protein was used directly for experiments without freezing.

### 1.2 Immunization experiment for EBV R9AP rabbit

### 1.2.1 Vaccine preparation

(1) The GST-R9AP protein was thawed after taking out from the -80°C freezer. A dose of 50 µg per rabbit was used. An antigen solution was prepared using sterile phosphate buffered saline (PBS) as the solvent.
(2) 500 µL of the antigen solution was mixed with an equal volume of 500 µL of Complete Freund's Adjuvant (CFA). The mixture was emulsified to form a dose of 1 mL per rabbit, and was placed on an inversion shaker at 4 °C to mix overnight.

### 1.2.2 Rabbit immunization and spleen collection

(1) Rabbits were fed with conventional feed and conventional drinking water. Immunization was performed at 12 weeks of age, and the strain used was New Zealand White rabbit.
(2) The rabbit was fixed. The manually re-emulsified vaccine was aspirated with a 2 mL syringe, and then administered via intramuscular injection into the leg.
(3) The rabbit was observed for 3-5 minutes, and was returned to its housing cage after normal activity resumed.
(4) The vaccine immunization schedule was performed as follows: week 0, week 3, week 6, and week 8. The spleen was collected at week 10.
(5) When collecting the rabbit spleen, according to animal welfare and standard animal experimental procedures, the rabbit was sacrificed by cervical dislocation. Its blood was collected, and its spleen was obtained by dissection.

### 1.3 Isolation and cloning of EBV R9AP rabbit B cell R9AP-specific cell

### 1.3.1 Spleen lymphocyte isolation steps:

(1) At week 10 after immunization, the rabbit was sacrificed by cervical dislocation. Its peripheral lymph nodes and spleen were isolated and placed into an EP tube.
(2) Appropriate amounts of DNase and type IV collagenase were added to 1640 medium to prepare a tissue digestion solution. An appropriate amount of digestion solution was added according to the size of the lymph nodes and spleen.
(3) The tissue was placed on a 70 µm cell strainer. A sterile syringe plunger was used to crush the tissue until it was almost fragmented. Then the cell strainer was rinsed with an appropriate amount of fresh medium to collect all remaining cells into a 50 mL centrifuge tube below.
(4) Red blood cell lysis buffer was added, and a resulting mixture was incubated at room temperature for 10 minutes.
(5) Centrifugation was performed at 2000g, 18 °C for 5 minutes, and the supernatant was discarded.
(6) The cells were washed twice with fresh medium.

### 1.3.2 Staining and sorting of B lymphocyte antigen

(1) The cells were washed with PBS and adjusted to 1×10⁸ cells per flow tube. Centrifugation was performed at 3000g, 18 °C for 5 minutes, and the supernatant was discarded.
(2) 5 µL of Fc receptor blocking antibody solution was added to 100 µL of PBS. The cells were resuspended and incubated on ice for 30 minutes.
(3) Antibody diluent for flow cytometry was prepared with PBS according to the cell groupings. When the antibody was used for the first time, different dilution gradients were set. Tests were performed at ratios of antibody: PBS of 1:20, 1:50, 1:100, 1:200, and 1:500, and the optimal gradient was selected. 100 µL of the diluted antibody for flow cytometry was mixed with dye for cell viability. The cells were resuspended for staining and were incubated at 4 °C in the dark for 30 minutes. Rabbit IgG and B220 antibodies were used as B cell isolation reagents, and R9AP was used as the R9AP-specific B cell isolation reagent.
(4) The cells were washed with PBS. Centrifugation was performed at 3000g, 4 °C for 5 minutes. The washing was repeated 1-2 times, and the supernatant was discarded.
(5) The cells were resuspended in PBS into flow cytometry tubes, and were sorted by flow cytometry using a BD Rhapsody single-cell sorter.
(6) The 0.2% of cells with the strongest R9AP positive signal among the IgG⁺B220⁺ cells were sorted into a 96-well plate.

### 1.3.3 Cloning and amplification of B cell sequence

(1) PCR was performed on B cells in the 96-well plate using rabbit IgG heavy chain and light chain universal cloning primers to obtain the heavy chain and light chain sequences of the corresponding single B cell.
   The forward (F) and reverse (R) primer sequences for the heavy chain V_{H} are as follows:
   F: ggtggttcctctagatcttcctcctctggtggcggtggctcgggcggtggtgggCAGTCGBTGGAGGAGT (SEQ ID NO: 22).
   R: gctggccggcctggccactagtTGARGAGACGGTGACC (SEQ ID NO: 23).
   The forward (F) and reverse (R) primer sequences for the light chain V_{L} are as follows:
   F: ggtggttcctctagatcttcctcctctggtggcggtggctcgggcggtggtgggCAGTCGBTGGAGGAGT (SEQ ID NO: 24).
   R: gctggccggcctggccactagtTGARGAGACGGTGACC (SEQ ID NO: 25).
(2) The upstream of heavy chain variable region of the antibody was connected to a cytomegalovirus (CMV) fragment, and the downstream was connected to the constant region of rabbit IgG1, so that a recombinant fragment capable of expressing the complete heavy chain was constructed; whereas the upstream of light chain variable region of the antibody was connected to a CMV fragment, and the downstream was connected to the constant region of light chain κ/λ, so that a recombinant fragment capable of expressing the complete light chain was constructed. Plasmids containing the full-length sequences of the above antibody heavy chain and light chain were co-transfected into 293F cells, whereby the expression of the antibody was achieved. The antibody was purified using protein A beads. A total of 9 antibodies were obtained by this method.
(3) R9AP affinity determination of the obtained antibodies was performed using enzyme-linked immunosorbent assay (ELISA). The 5E9 antibody with the highest affinity was screened out and was used for further validation.

### Example 2: Preparation of anti-human R9AP protein monoclonal antibody 5E9 (mAb)

The upstream of heavy chain variable region of the antibody was connected to a CMV fragment, and the downstream was connected to the constant region of human IgG1, so that a recombinant fragment capable of expressing the complete heavy chain was constructed; whereas the upstream of light chain variable region of the antibody was connected to a CMV fragment, and the downstream was connected to the constant region of light chain κ/λ, so that a recombinant fragment capable of expressing the complete light chain was constructed. Plasmids containing the full-length sequences of the above antibody heavy chain and light chain were co-transfected into 293F cells, whereby the expression of the antibody was achieved. The antibody was purified using protein A beads.

The 5E9 heavy chain consists of 128 amino acid residues, with the specific sequence as follows: 
METGLRWLLLVAVLKGVQCQSLEESGGRLVTPGTPLTLSCKASGFDFSSYWMIWVRQ
APGEGLEYIGIITKSGNTYYANWAKGRFTISKTSTTVDLKITSPTTEDTATYFCTSNFKLWGQGT
LVTVSS (SEQ ID NO: 3);
 wherein the underlined part of the sequence is the amino acid sequence of the heavy chain variable region (SEQ ID NO: 4). The underlined and italicized parts are sequentially the amino acid sequences of the three complementarity regions CDR-H1 (*SYWMI*, SEQ ID NO: 5), CDR-H2 (*IITKSGNTYYANWAKG*, SEQ ID NO: 6), and CDR-H3 (*NFKL*, SEQ ID NO: 7) in the heavy chain variable region (IMGT definition scheme). The bold part is the signal peptide sequence. * indicates a stop codon.

The sequences of CDR-H1, CDR-H2, CDR-H3 in this heavy chain variable region using other CDR definition schemes are shown in Table 11.

**Table 11: Sequences of CDR-H1, CDR-H2, CDR-H3 in the heavy chain variable region using other CDR definition schemes**

| Other CDR definition scheme | CDR-H1 | CDR-H2 | CDR-H3 |
|---|---|---|---|
| Kabat | SYWMI (SEQ ID NO: 5) | IITKSGNTYYANWAK G (SEQ ID NO: 6) | NFKL (SEQ ID NO: 7) |
| Chothia | GFDFSSY (SEQ ID NO: 8) | TKSGN (SEQ ID NO: 9) | GGYRYDSDY (SEQ ID NO: 10) |
| Contact | SSYWMI (SEQ ID NO: 11) | YIGIITKSGNTY (SEQ ID NO: 12) | TSNFK (SEQ ID NO: 13) |

The 5E9 light chain variable region consists of 132 amino acid residues, with the specific sequence as follows: 
MDTRAPTQLLGLLLLWLPGATFAQVLTQTASPVSAAVGGTVTINCQSSQSVYNNNNL
AWFQQKPGQPPKVLITSASTLASGVPSRFSGSGSGTQFTLTISDLECDDAAAYYCAGGYSGNIY
TFGGGTDVVVK (SEQ ID NO: 14);
 wherein the underlined part of the sequence is the amino acid sequence of the light chain variable region (SEQ ID NO: 15). The underlined and italicized parts are sequentially the amino acid sequences of the three complementarity determining regions CDR-L1 (*QSSQSVYNNNNLA*, SEQ ID NO: 16), CDR-L2 (*SASTLAS*, SEQ ID NO: 17), and CDR-L3 (*AGGYSGNIYT*, SEQ ID NO: 18) in the light chain variable region (IMGT definition scheme). The bold part is the signal peptide sequence. * indicates a stop codon.

The sequences of CDR-L1, CDR-L2, CDR-L3 in this light chain variable region using other CDR definition schemes are shown in Table 12.

**Table 12: Sequences of CDR-L1, CDR-L2, CDR-L3 in the light chain variable region using other CDR definition schemes**

| Other CDR definition scheme | CDR-L1 | CDR-L2 | CDR-L3 |
|---|---|---|---|
| Kabat | QSSQSVYNNNNLA (SEQ ID NO: 16) | SASTLAS (SEQ ID N O: 17) | AGGYSGNIYT (SEQ ID NO: 18) |
| Chothia | QSSQSVYNNNNLA (SEQ ID NO: 16) | SASTLAS (SEQ ID N O: 17) | AGGYSGNIYT (SEQ ID NO: 18) |
| Contact | YNNNNLAWF (SEQ ID NO: 19) | VLITSASTLA (SEQ ID NO: 20) | AGGYSGNIY (SEQ ID NO: 21) |

### Example 3: Affinity determination of 5E9

### 3.1 Detection of 5E9 binding ability using ELISA

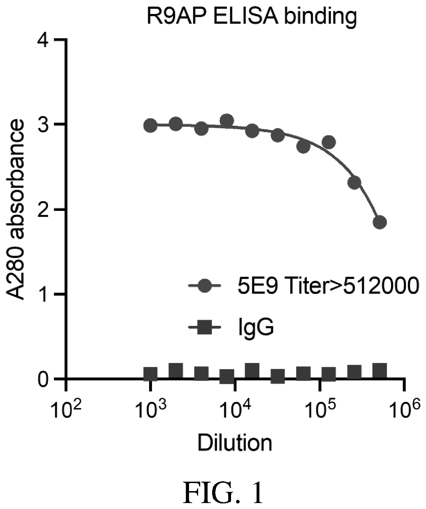

ELISA could be performed according to conventional methods in the art. In this example, the specific operations were as follows: R9AP protein was dissolved in a PBS solution at a concentration of 10 µg/mL, and added to an ELISA high-binding plate at 100 µL per well. Primary coating was carried out at room temperature for 3 hours, after which the liquid was discarded. A 0.2 mg/mL BSA solution was prepared, added to the ELISA plate, and well blocking was performed at room temperature for 6 hours, after which the liquid was discarded. Subsequently, the 5E9 antibody was serially diluted starting from 1:10, added to the ELISA plate, and primary binding was performed at room temperature for 2 hours, after which the liquid was discarded. Then, a 1:5000 diluted goat anti-rabbit IgG-HRP secondary antibody was prepared, added to the ELISA plate, and secondary binding was performed at room temperature for 30 minutes, after which the liquid was discarded. Finally, TMB substrate was added, color development was carried out at room temperature for 15 minutes, and the color development was stopped using dilute hydrochloric acid. The A280 reading of each well was measured using a microplate reader, which served as an indicator of the binding ability of R9AP antibody in each well, with a higher reading indicating a stronger binding strength. The results are shown in FIG. 1: the A280 reading of the 5E9 antibody remain at a high binding level even at a 1:512,000 dilution, while the control of irrelevant IgG antibody shows no binding ability to R9AP at all, demonstrating that the 5E9 antibody has a high binding ability to R9AP.

### 3.2 Determination of the affinity of antibody 5E9 using Bio-Layer Interferometry (BLI)

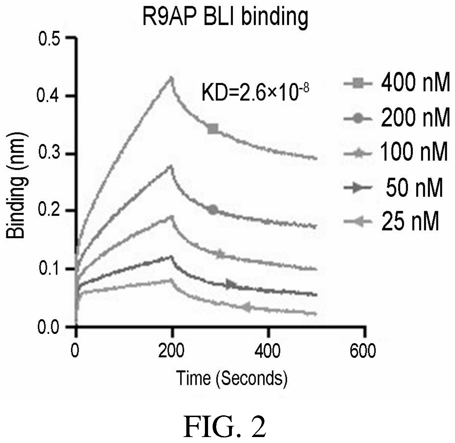

BLI could be performed according to conventional methods in the art. In this example, the specific operations were as follows: A biosensor (Sartorius Octet^{®} SA probe, Sartorius, Germany) was immersed in a buffer (a mixture of KB buffer, 0.1 wt% BSA, and 0.02 v/v% Tween 20) for equilibration. Then, the biosensor was removed and immersed in a solution containing 5 µg/mL R9AP-Biotin (biotin-labeled R9AP protein). The R9AP antigen in the solution was bound to the surface of the streptavidin (SA) biosensor probe, which caused an increase in the thickness of surface film. Then the biosensor with the immobilized antigen at a known concentration was immersed in the buffer, and the baseline signal was recorded. By immersing the biosensor with the immobilized antigen at a known concentration into a sample solution containing 31.3-500 nM 5E9 antibody for about 120 seconds, the film thickness was increased due to the specific antigen-antibody binding. The biosensor that had bound the 5E9 antibody was immersed in the buffer for dissociation for about 180 seconds. The test antibody (5E9 antibody) was dissociated from the biosensor surface, and the film thickness was decreased. Through real-time monitoring of the thickness of biosensor's biofilm during the experiment, the kinetic constant of the test sample (5E9 antibody) was obtained. The results are shown in FIG. 2: the KD (M) of the 5E9 antibody is 2.6×10⁻⁸ M, indicating that the 5E9 antibody has a high affinity for the R9AP antigen.

### 3.3 Determination of the specific binding ability of antibody 5E9 to R9AP using Western-Blot

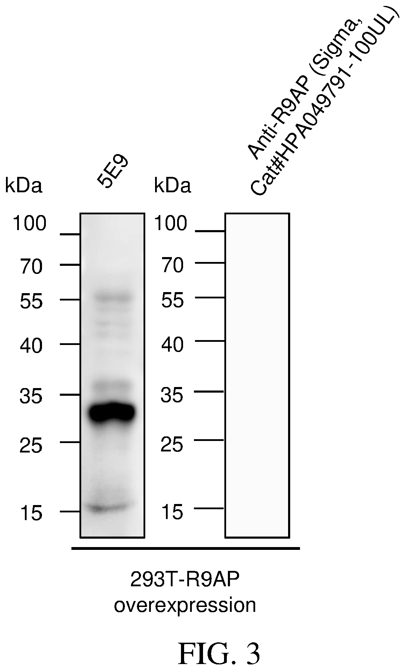

Western blot (WB) could be performed according to conventional methods in the art. In this example, the specific operations were as follows: The full-length R9AP plasmid was overexpressed in 293 cells using PEI. Subsequently, the cells were lysed with WB lysis buffer. After thorough mixing, the fully lysed cell contents were collected, and the precipitate was removed by centrifugation. Then, the protein samples were separated by SDS-PAGE and were transferred onto a 0.45 µm PVDF membrane using an electroblotting apparatus. Then, the 5E9 antibody and the R9AP positive antibody (Sigma, catalog #HPA049791-100UL) were respectively added onto the PVDF membrane, incubation and binding were carried out at 4 °C for 8 hours, after which the antibodies were discarded. Subsequently, a 1:2000 diluted goat anti-rabbit IgG-HRP secondary antibody and a 1:4000 diluted rabbit anti-mouse IgG-HRP secondary antibody were respectively added, incubated at room temperature for 30 minutes, after which the antibodies were discarded. Finally, horseradish peroxidase substrate was added for color development, and images were taken using a BioRad illuminator. The results are shown in FIG. 3: the 5E9 antibody shows a band position consistent with that of the fully validated R9AP antibody from Sigma for specific binding to R9AP, indicating that the 5E9 antibody has specific binding to the R9AP antigen.

### Example 4: Neutralization activity assay of 5E9 antibody

### (1) Preparation of EBV virus:

1) CNE2 cells infected with EBV-GFP (the virus was disclosed in the literature: "An Antibody Targeting the Fusion Machinery Neutralizes Dual-Tropic Infection and Defines a Site of Vulnerability on Epstein-Barr Virus") were cultured in RPMI1640 medium supplemented with 5 v/v% FBS in a 37 °C incubator (5 v/v% CO₂). When the cell density reached 90% (10 cm dish), phorbol-12-myristate-13-acetate (TPA or PMA) at a final concentration of 20 ng/mL and sodium butyrate (NaB) at a final concentration of 2.5 mM were added for induction. The medium was changed after 12 hours.
2) At 48-72 hours after the medium change, the culture supernatant was collected, and the virus was isolated and purified. The supernatant was directly aspirated, centrifuged, and filtered through a 0.45 µm small filter. The filtrate was concentrated, resuspended in serum-free RPMI1640, and then immediately used for infection or was stored at -80 °C.

### (2) Neutralization activity assay of the 5E9 monoclonal antibody on epithelial cells

1) 1×10⁶ 293T epithelial cells were seeded per well in a 96-well plate, and 100 µL of DMEM medium containing 10% FBS was added to each well.
2) On the next day, the 5E9 monoclonal antibody from the above example was adjusted to a concentration of 2 mg/mL. In a new 96-well plate, 60 µL of DMEM medium was added to each well. 120 µL of 12.5 µg/mL 5E9 antibody diluted with DMEM was added to the first well (the first well did not contain RPMI1640 medium).
3) After 2-fold serial dilution (the serial dilution procedure was as follows: 60 µL was aspirated from the first well and added to the second well, and so on; 60 µL was aspirated from the last well and discarded; a final volume per well was 60 µL), 60 µL of virus diluent (the virus was diluted with DMEM medium to a titer of approximately 4×10⁶/mL) was added to each well, incubated at 37 °C for 2 hours, and a resulting mixture was added to the 293T cells that had been plated the previous day. The cells were placed in a 37 °C incubator and cultured for 48 hours for the assay.
4) The 293T cells were digested with trypsin to prepare a cell suspension. The infection rate was detected by flow cytometry. By measuring the reduction ratio in the number of GFP-positive cells in the antibody-treated group compared to the infection control group (to which an equal volume of DMEM was added), the inhibition rate (neutralization efficiency, %) of the antibody in the 293T epithelial cell infection model was calculated.

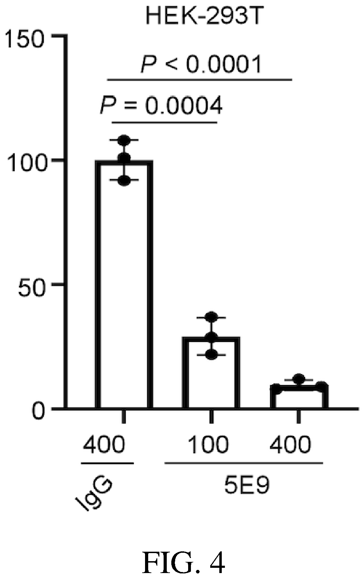

The results are shown in FIG. 4: the monoclonal antibody 5E9 at a concentration of 400 µg/mL can significantly inhibit EBV infection of epithelial cells, wherein the vertical axis represents the EBV infection efficiency.

### Example 5: Neutralization activity assay of 5E9 monoclonal antibody on B cells

1) 5E9 monoclonal antibody from the above example was adjusted to a concentration of 2 mg/mL. In a new 96-well plate, 60 µL of RPMI1640 medium was added to each well. 90 µL of 100 µg/mL 5E9 antibody diluted with RPMI1640 was added to the first well (the first well did not contain RPMI1640 medium).
2) After 3-fold serial dilution (the serial dilution procedure was as follows: 30 µL was aspirated from the first well and added to the second well, and so on; 30 µL was aspirated from the last well and discarded; a final volume per well was 60 µL), 60 µL of virus diluent (the virus was diluted with DMEM medium to a titer of approximately 4×10⁶/mL) was added to each well, incubated at 37 °C for 2 hours, and then 1×10⁶ Raji cells were added per well. The cells were placed in a 37 °C incubator and cultured for 48 hours for the assay.
3) The Raji cells were aspirated to prepare a cell suspension. The infection rate was detected by flow cytometry. By measuring the reduction ratio in the number of GFP-positive cells in the antibody-treated group compared to the infection control group (to which an equal volume of RPMI1640 was added), the inhibition rate (neutralization efficiency, %) of the antibody in the Raji B cell infection model was calculated.

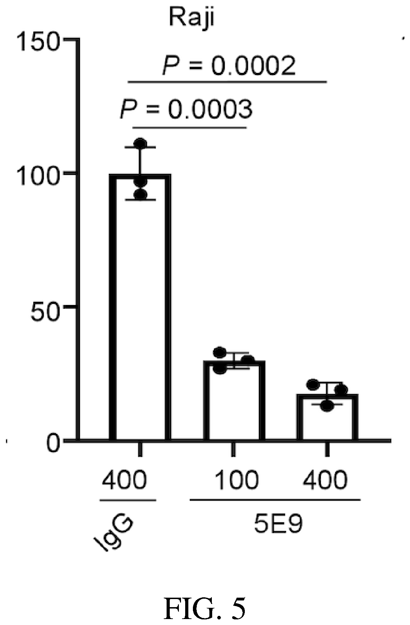

The results are shown in FIG. 5: the monoclonal antibody 5E9 at a concentration of 400 µg/mL can significantly inhibit EBV infection of B cells in the B cell infection model, wherein the vertical axis represents the EBV infection efficiency.

## Claims

1. A monoclonal antibody or an antigen-binding fragment thereof against human R9AP protein, wherein the monoclonal antibody or the antigen-binding fragment thereof comprises a heavy chain variable region and a light chain variable region;
the heavy chain variable region comprises CDR-H1, CDR-H2, and CDR-H3;
the CDR-H1, CDR-H2, and CDR-H3 are CDR1, CDR2, CDR3 of an amino acid sequence shown in SEQ ID NO: 4;
the light chain variable region comprises CDR-L1, CDR-L2, and CDR-L3;
the CDR-L1, CDR-L2, and CDR-L3 are CDR1, CDR2, CDR3 of an amino acid sequence shown in SEQ ID NO: 15.

2. The monoclonal antibody or the antigen-binding fragment thereof according to claim 1, wherein amino acid sequences of the CDR-H1, CDR-H2, CDR-H3, CDR-L1, CDR-L2, CDR-L3 are sequentially as shown in SEQ ID NO: 5, SEQ ID NO: 6, SEQ ID NO: 7, SEQ ID NO: 16, SEQ ID NO: 17, and SEQ ID NO: 18, and the CDRs are defined by an IMGT definition scheme; or
the amino acid sequences of the CDR-H1, CDR-H2, CDR-H3, CDR-L1, CDR-L2, CDR-L3 are sequentially as shown in SEQ ID NO: 5, SEQ ID NO: 6, SEQ ID NO: 7, SEQ ID NO: 16, SEQ ID NO: 17, and SEQ ID NO: 18, and the CDRs are defined by a Kabat definition scheme; or
the amino acid sequences of the CDR-H1, CDR-H2, CDR-H3, CDR-L1, CDR-L2, CDR-L3 are sequentially as shown in SEQ ID NO: 8, SEQ ID NO: 9, SEQ ID NO: 10, SEQ ID NO: 16, SEQ ID NO: 17, and SEQ ID NO: 18, and the CDRs are defined by a Chothia definition scheme; or
the amino acid sequences of the CDR-H1, CDR-H2, CDR-H3, CDR-L1, CDR-L2, CDR-L3 are sequentially as shown in SEQ ID NO: 11, SEQ ID NO: 12, SEQ ID NO: 13, SEQ ID NO: 19, SEQ ID NO: 20, and SEQ ID NO: 21, and the CDRs are defined by a Contact definition scheme.

3. The monoclonal antibody or the antigen-binding fragment thereof according to claim 1 or 2, wherein the monoclonal antibody or the antigen-binding fragment thereof comprises at least one of a full-length antibody, Fab, Fab', F(ab')2, Fv, scFv, a bispecific antibody, or a multispecific antibody.

4. A recombinant protein, comprising the monoclonal antibody or the antigen-binding fragment thereof according to any one of claims 1-3; and optionally a tag sequence that assists expression and/or purification.

5. A biological material related to the monoclonal antibody or the antigen-binding fragment thereof according to any one of claims 1-3, or the recombinant protein according to claim 4, wherein the biological material comprises at least one of h1) to h16):
h1) a nucleic acid molecule encoding the monoclonal antibody or the antigen-binding fragment thereof according to any one of claims 1-3, or the recombinant protein according to claim 4;
h2) an expression cassette comprising the nucleic acid molecule of h1);
h3) a vector comprising the nucleic acid molecule of h1);
h4) a vector comprising the expression cassette of h2);
h5) a transgenic cell line comprising the nucleic acid molecule of h1);
h6) a transgenic cell line comprising the expression cassette of h2);
h7) a transgenic cell line comprising the vector of h3);
h8) a transgenic cell line comprising the vector of h4);
h9) a microorganism comprising the nucleic acid molecule of h1);
h10) a microorganism comprising the expression cassette of h2);
h11) a microorganism comprising the vector of h3);
h12) a microorganism comprising the vector of h4);
h13) a virus comprising the nucleic acid molecule of h1);
h14) a virus comprising the expression cassette of h2);
h15) a virus comprising the vector of h3);
h16) a virus comprising the vector of h4).

6. A method for preparing the monoclonal antibody or the antigen-binding fragment thereof according to any one of claims 1-3, or the recombinant protein according to claim 4, comprising the following steps: expressing and then purifying the biological material according to claim 5 to obtain the monoclonal antibody or the antigen-binding fragment thereof or the recombinant protein.

7. A conjugate comprising at least one of the monoclonal antibody or the antigen-binding fragment thereof according to any one of claims 1-3, or the recombinant protein according to claim 4; and a conjugated moiety, wherein the conjugated moiety comprises at least one of a detectable label, a drug, a toxin, a cytokine, a radionuclide, or an enzyme.

8. Use of at least one of (1) to (4) in the preparation of a product:
(1) the monoclonal antibody or the antigen-binding fragment thereof according to any one of claims 1-3;
(2) the recombinant protein according to claim 4;
(3) the biological material according to claim 5;
(4) the conjugate according to claim 7;
wherein the product comprises at least one of a drug, a vaccine, a reagent, a detection plate, a kit, or a detection chip.

9. A drug, comprising at least one of (11) to (14):
(11) the monoclonal antibody or the antigen-binding fragment thereof according to any one of claims 1-3;
(12) the recombinant protein according to claim 4;
(13) the biological material according to claim 5;
(14) the conjugate according to claim 7;
preferably, the drug further comprises a pharmaceutically acceptable carrier.

10. A kit for detecting Epstein-Barr virus, wherein the kit comprises at least one of (11) to (14) and an adjuvant:
(11) the monoclonal antibody or the antigen-binding fragment thereof according to any one of claims 1-3;
(12) the recombinant protein according to claim 4;
(13) the biological material according to claim 5;
(14) the conjugate according to claim 7.
